# EUROPEAN PATENT SPECIFICATION

(11) **EP 1 972 270 B1**
(45) Date of publication and mention of the grant of the patent: **03.11.2010**
(21) Application number: 07405101.2
(22) Date of filing: 23.03.2007
(51) Int. Cl.: A61B 5/00, G01N 33/66, G06F 19/00

(54) **Method and glucose monitoring system for monitoring individual metabolic response**
Verfahren und Glukoseüberwachungssystem zur Überwachung individueller Stoffwechselreaktionen
Procédé et système de surveillance du glucose pour la surveillance de la réponse métabolique individuelle

(43) Date of publication of application: 24.09.2008
(73) Proprietor: Roche Diagnostics GmbH, 68305 Mannheim (DE); F.Hoffmann-La Roche AG, 4070 Basel (CH)
(72) Inventor: Heaton, Kelly, 3423 Ersigen (CH)
(74) Representative: Rüfenacht, Philipp Michael

(56) References cited:
- WO-A-00/18293
- WO-A-2006/066926
- WO-A2-2004/043230
- US-A1- 2004 225 205
- US-A1- 2005 244 910

## Description

### Technical Field

The invention relates to a method for monitoring individual metabolic response as well as to a glucose monitoring system for carrying out the method. The invention further relates to a computer program product for carrying out the inventive method.

### Background Art

After ingestion of food or beverages that contain carbohydrates, these carbohydrates are broken down during digestion and thereby converted to mono- and disaccharides, mostly glucose. Glucose is a source of energy for the cells of the organism. This energy is yielded within the cells through glycolisis and subsequent reactions of the Citric acid cycle. Glucose is transported to the cells via the organism's blood stream. Therefore, ingestion of food will have an influence on the concentration of glucose within the blood stream; i. e. the blood glucose level will change. This effect, more precisely the rate at which the ingested food or beverage is able to increase the blood glucose level and the length of time the blood glucose remains elevated, is denoted by the term "glycemic response".

There is significant evidence that a calorie-restricted diet promotes good health (U. S. National institute on Aging, Primate Aging Study).

One benefit of a calorie-restricted diet is an overall reduction of blood glucose levels. High blood glucose concentrations have been correlated with numerous health problems including oxidative stress, micro- and macro-vascular tissue damage, heart disease, hypertension and Type 2 diabetes. Extreme fluctuations in blood glucose have also been shown to stimulate appetite, an undesirable experience for dieters struggling to avoid hunger pangs (see e. g. US 6,905,702, Los Angeles Children's Hospital).

Humans are recommended to receive between 45 - 65% of daily caloric intake from carbohydrates, which are metabolized by the body as sugars (Reference: Dietary Guidelines for Americans, 2005) and measurable as blood glucose concentration following a meal. Certain carbohydrate-containing foods are rapidly absorbed into the blood stream, causing a rapid increase of blood glucose levels and acutely over-supplying the body with energy. Habitual consumption of rapidly absorbed carbohydrates, especially in large quantities, is one of the primary drivers of Metabolic Syndrome and Type 2 diabetes. Preferably, a meal will release carbohydrates into the blood slowly, producing a gradual rise that is manageable by the body's tissues.

Established metrics to evaluate the glycemic impact of carbohydrate-containing foods are the Glycemic Index (GI) and the Glycemic Load (GL). The Glycemic Index (GI) is proportional to the area under the curve (AUC) when blood glucose concentration is plotted against time, wherein only the two hours following the ingestion of a fixed portion of carbohydrate (usually 50 g) are considered. The AUC of the test food is divided by the AUC of a reference food portion (either glucose or white bread) of equal carbohydrate content and multiplied by 100. The average GI value is calculated from data collected in a sample population and is available in GI tables (e. g. J. Brand-Miller, K. Foster-Powell, "Shopper's Guide to GI Values", Marlowe & Company, 2007). Glycemic Load (GL) takes into account the portion size of the ingested food. It is calculated as the quantity (in grams) of its carbohydrate content, multiplied by its GI, and divided by 100.

Both GI and GL indices have been recommended for use in food labeling, with partial acceptance in the field of nutrition. The two major criticisms of GI and GL are: (1) GI and GL cannot be used to predict glycemic response for foods eaten in combination (and therefore cannot be used to compute the glycemic impact of a mixed meal); and (2) the individual response to a food of known GI and GL will vary considerably due to multiple factors, such as weight, gender, and genetic factors (G. Ruano et al. "Physiogenomic analysis of weight loss induced by dietary carbohydrate restriction.", Nutrition and Metabolism 2006, 3:20).

Chemical interactions between food items have a highly significant impact on the behavior of carbohydrate release into the bloodstream. Studies indicate that when several foods of known GI are mixed in a meal, the individual's glycemic response is often unpredictable; although most of the glucose in the blood stream originates from ingested carbohydrates other foodstuffs such as proteins, fat or food fibres may substantially affect the release and absorption of glucose and also pancreatic and liver function (Hollenbeck, CB et al. "The clinical utility of the glycemic index and its application to mixed meals.", Can J Physiol Pharmacol 69:100-107, 1991). The portion of food consumed also plays a significant role on its glycemic effect, and is not linearly correlated with the effect of the food on glycemic response. Finally, the glycemic effect of "same" food items, such as "a potato," will vary according to size, age, growing season and region, cooking time, etc. Therefore, GI and GL are often poor predictors of individual response for even isolated foods and mixed meals are nearly impossible to model.

The US patent application No. 2004/0043106 A1 (J. R. Anfinsen et al.) addresses item (1) of the criticisms mentioned above. The document discloses methods for establishing the Equivalent Glycemic Load (EGL) of food products as well as systems for selecting food products by consumers for the management of their intake of foods that elicit glycemic responses. For determining the EGL of a dietary comestible, first the glycemic response produced by that comestible is determined. The standard comestible Glycemic Load which is correlated with this glycemic response is identified from the index. Such load is the standard comestible EGL of the dietary comestible. In the context of the disclosed methods, the dietary comestible cannot only be a single food product but it can comprise more than one food product, i. e. correspond to a mixed meal. Based on the EGL, a personalized diet may be created. Again, this diet method is based on standardized food labeling.

Recapitulating, glycemic response is important to anyone who wants to lose or gain weight; avoid heart disease, metabolic syndrome, hypertension or Type 2 diabetes; and anyone concerned with optimizing athletic performance, cf. "The New Glucose Revolution: The Authoritative Guide to the Glycemic Index - The Dietary Solution for Lifelong Health" (published by Marlow & Company), by Dr. Jennie Brand-Miller, Dr. Thomas M. S. Wolever, Kaye Foster-Powell, Dr. Stephan Colagiuri.

However, inter- as well as intra-individual variation on the individual response to a foodstuff is not addressed by the methods mentioned above. On the contrary, all labeled food items are assumed to have the same effect on the blood glucose level of any consumer.

Furthermore, the act of calorie counting or calculating and summing up EGL or GL values can be frustrating, tedious and imprecise for people who are trying to manage their weight. Many people struggle to manage their weight because they wrongly estimate the metabolic effects of habitual food choices. Other people fail to accurately measure their food portions. This is especially true in everyday situations, such as restaurant meals, travel or casual meals at home or with friends. In these situations, calculating a diet based on food labels is not desirable or even not feasible.

Therefore, instead of having to rely on tabulated averages relating to the metabolism of an average organism and on assumptions about the content and size of food portions it would be desirable for an individual to be able to rely on their actual, individual metabolic response to an actual meal consumed.

WO 2004/043230 relates to a method of screening for disorders of the glucose metabolism such as diabetes or impaired glucose tolerance. For this purpose, a mathematical algorithm evaluates the shape of a subject's blood glucose profile before and after a glucose challenge and classifies the profile into one of several predefined classes, each class corresponding either to a normal condition or one of several abnormal conditions. Evaluation of the shape of the profile is accomplished through examination of one or more parameters of the profile and may involve simple comparisons between parameters to established thresholds or ranges or calculating further characteristics such as a screening factor. The time series of blood glucose concentrations may be measured by any type of glucose analyzer; the values may be absolute or relative values. Depending on the outcome of the screening, a subject may be provided with additional information concerning their condition. The collection rate of glucose measurements may be collected once every ten to sixty minutes. Two of the proposed parameters are the area above a normal glucose baseline during the course of a glucose tolerance test, and the area under the curve after the peak glucose concentration to an endpoint in time, respectively.

### Summary of the invention

Therefore, it is the object of the invention to create a system and method for monitoring individual metabolic response pertaining to the technical field initially mentioned, that is comfortable for the user and that provides a personalized and specific feedback supporting the user's dietary management.

The solution of the invention is specified by the features of claims 1 and 12. According to the invention the method for monitoring individual metabolic response and for generating nutritional feedback involves monitoring of glycemic response in a qualified subject, comprising the steps of:
a) consecutively performing a plurality of measurements of a glucose level in the subject by a measuring device;
b) in the measuring device generating first data corresponding to the measured glucose level;
c) transmitting the first data to an analysis device;
d) in the analysis device generating second data representing a glycemic response of the subject involving comparing a time-series of glucose measurements represented by the first data with a reference value for the fasting glucose level of the subject;
e) comparing the second data with a predetermined individual glycemic response budget for the qualified subject, the individual glycemic response budget representing a total amount of individual glycemic response allowable for a certain time period corresponding to a recommended total carbohydrate intake during this time period; and
f) providing feedback corresponding to a result of the comparison on an output device of the second data with the predetermined individual glycemic response budget.

Correspondingly, a glucose monitoring system for monitoring glycemic response in a qualified subject and for generating nutritional feedback, comprises
a) a measuring device comprising a sensor for consecutively performing a plurality of measurements of a glucose level in the qualified subject and comprising a data generator for generating first data corresponding to the measured glucose level;
b) an analysis device comprising a computer to generate second data from the first data, the second data representing a glycemic response of the subject and the generation of the second data involving comparing a time-series of glucose measurements represented by the first data with a reference value for the fasting glucose level of the subject, and to compare the second data with a predetermined individual glycemic response budget for the qualified subject, the individual glycemic response budget representing a total amount of individual glycemic response allowable for a certain time period corresponding to a recommended total carbohydrate intake during this time period; and
c) a computer-controlled output device to provide feedback corresponding to a result of the comparison of the second data with the predetermined individual glycemic response budget.

Usually, steps d) and e) of the inventive method will be carried out by a computer program product that includes program code which when executed on an analysis device carries out the following steps:
a) generating second data representing a glycemic response of a qualified subject from first data, involving comparing a time-series of glucose measurements in the qualified subject, represented by the first data, with a reference value for the fasting glucose level of the subject;
b) comparing the second data with a predetermined individual glucose response budget for the qualified subject, the individual glucose response budget representing a total amount of individual glycemic response allowable for a certain time period corresponding to a recommended total carbohydrate intake during this time period; and
c) generating at least one quantity relating to nutritional feedback based on the result of the comparison of the second data with the predetermined individual glycemic response budget.

In the context of the invention, a subject is qualified if it has a stable fasting glucose level without having to use exogenous insulin, i. e. a qualified subject is a human or animal who possesses the natural ability to metabolize carbohydrates without the use of exogenous insulin (in contrast to people suffering Type 1 diabetes, for example). This allows for obtaining meaningful results from the comparison of the second data with the predetermined individual glycemic response budget, and to draw meaningful conclusions concerning the subject's metabolic response to carbohydrates consumed.

In the context of the invention, suitable frequencies for the consecutive measurements of the glucose level start from at least 1 measurement an hour and range in particular from 4 to 60 measurements an hour. The glucose level may be measured in any measurable tissue compartment, e. g. in blood or interstitial fluid. In principle, non-invasive methods of glucose monitoring are also acceptable in the context of this invention, as soon as a sufficient measuring accuracy can be achieved. The measuring device and/or the analysis device and/or the output device may be integrated into one single unit or they may be comprised by different units or even distributed to several units.

The generation of the first data involves converting the output of the actual sensor component into a signal (e. g. an analog or digital signal in the form of a voltage) that may be transmitted to the analysis device. The first data generation may involve further conversion of the sensor output. The generation step may happen within a usual sensor device or within a circuit or computing unit of the measuring device.

The glycemic response of the subject is comprised of a time-series of the measured glucose values documenting the temporal rise, fall or stasis of the subject's physiological glucose levels, comprising at least two, preferably at least three measurements taken at different times. The individual glycemic response is obtained by comparing the time-series of glucose measurements represented by the first data with a reference value for the fasting glucose level of the subject. This reference value may be measured by employing usual methods known from the prior art, e. g. by spot blood glucose measurements, and entered into the device by the user, its healthcare provider or nutritionist, or it may be established in the context of the inventive method or by the inventive monitoring system, respectively (see below).

The individual glycemic response may be represented as a series of numbers corresponding to the measured glucose concentrations, matched with the corresponding times of measurements. Alternatively, it may be represented as a plotted curve based on the measured time-glucose data pairs or as a calculated quantity, e. g. based on AUC or iAUC (see below). The second data representing the glycemic response of the subject is preferably stored in a storage of the analysis device. This storage may contain further user-specified information, e. g. relating to previous glycemic responses, the user's metabolic goals etc.

The individual glycemic response budget represents a total amount of individual glycemic response allowable for a certain time period. For the given time period it may consist of a single value or of a range, i. e. setting lower and upper target levels for desired glycemic response during the established time-period. Advantageously, the individual glycemic response budget is stored in a storage of the analysis device.

The individual glycemic response budget is established by a healthcare professional nutritionist, based on individual attributes of the subject and/or on medical/nutritional examinations of the subject. There are two preferred methods for calculating a glycemic response budget for healthy individuals with stable fasting-glucose levels. The first method is based on Thomas Wolever and Jennie Brand-Miller's method to calculate GI for a food item, adapted to measure individual response to pure glucose as the basis for calculating an individual glycemic response budget. It includes the following steps:
1. Following a fasting period of 8 or more hours, the subject's individual fasting glucose level is measured and recorded.
2. Subsequently, the subject consumes 50 grams of pure glucose.
3. The subject's individual glycemic response is recorded as a reference curve during 2 hours, whereas a measurement is taken at least every 15 minutes.
4. Taking into account the recorded fasting glucose level the measured response is converted into an AUC quantity (for example using the trapezoidal rule, taking into account the positive difference between the measured response and the fasting glucose level), hereafter referred to as the subject's individual reference glycemic response.
5. The subject's individual carbohydrate intake allowance (for 24 hours, for example) is estimated according to age, weight, height, gender, race, BMI, nutritional goals etc. of the individual. Ideally this calculation is performed by a doctor, a healthcare professional or a professional nutritionist.
6. The subject's individual carbohydrate intake allowance (in grams, for example) is used together with the individual reference glycemic response (see step 4) to calculate the subject's individual glycemic response budget for a defined period of time such as 24 hours. A sample calculation: a subject has an individual reference glycemic response of 100 (following the consumption of 50 grams of carbohydrates, see above, step 2) and a 24-hour recommended intake of carbohydrates of 400 grams. Therefore, the subject's 24-hour individual glycemic response budget is 800.

In the second method, the steps 1-4 of the first method are replaced by an estimation of a clinically established, average AUC value for human response to 50 g of pure glucose (adjusted for the subject's weight, gender, ethnicity, BMI, nutritional goals etc.). This reference value is assigned as the subject's individual reference glycemic response. Again, this calculation is preferably performed by a doctor, a healthcare professional or a professional nutritionist. After that, steps 5 and 6 of the first method described above follow. Further methods for calculating the individual glycemic response budget, based on individual attributes of the user and/or on medical/nutritional examinations of the user may be employed. Alternatively, the individual glycemic response budget may correspond to a prototype glycemic response representing an optimum progression of the glucose level or to a sample glycemic response that has been previously measured in the subject.

The comparison of the second data, representing the measured glycemic response of the subject, with a predetermined individual glycemic response budget for the subject allows for obtaining a conclusion relating to excess or insufficient consumption of carbohydrates, to weight gain or loss, hypertension, risk of Type 2 diabetes, chronic health problems etc.

The comparison may be based on absolute values (e. g. on an absolute difference between the individual glycemic response budget and the glycemic response) and/or on relative values (e. g. on the ratio or percentage of the individual glycemic response budget that has already been used by the glycemic response) or on any other suitable calculation rule.

The comparison may involve calculating an individual glycemic response budget for a time period that is shorter or longer than a time-interval for which the predetermined glycemic response budget has been established (e. g. 24 hours). In particular, this shorter or longer time period starts at a time of commencement of the accumulation of the glycemic response of the subject (i. e. at a time when the accumulated response is set back to 0) and ends at the current time. The calculation may be based on simple proportionality or it may involve more sophisticated mathematical and/or statistical methods. This allows for directly comparing the glycemic response with a budget value that corresponds to the current moment.

The inventive method and system will provide the subject with real-time information about their individual glycemic response as well as about their individual glycemic response budget and/or about the comparison between the two quantities. As an example, one or several of the following quantities may be displayed in text and/or graphical form:
a) value of the daily/weekly glycemic response budget (GRB);
b) value/fraction of the daily/weekly GRB that has already been "used";
c) remaining value/fraction of the daily/weekly GRB.

The numbers may be displayed e. g. as absolute values, ratios, fractions, decimal fractions or percentages. Instead of days or weeks other (meaningful) time intervals may be considered. Particularly, response budgets for single meals may be established, employed for comparisons and displayed. Instead of or additional to visual display all of the aforementioned information may be communicated by auditive output or other communication means.

It may be within the inventive method to take into account physical exercise by accordingly reducing the "used GRB" or by increasing the daily or weekly GRB depending on the duration and intensity of the physical exercise.

Continuously monitoring the glucose level of subjects having a stable fasting glucose level can provide highly personalized information about the effects of diet and nutrition by revealing an individual's glycemic response to ingested food and beverages. The inventive method and system are therefore valuable tools for supporting weight management, nutritional counseling and preventive medicine.

Monitoring the glucose level is also an excellent method for revealing the metabolic effects of exercise, an important aspect of any weight management or healthcare program. The benefits of recording the personal response to diet and exercise are numerous, including improved knowledge of the individual's body, improved insulin sensitivity, motivation and learning the effects of behavior, especially concerning food choices, on the individual's metabolism. For dieters, athletes (competitive as well as recreational) that aim at improving their athletic performance, or other people that have to take care of their blood glucose levels (such as people with obesity, hypertension, or risk of diabetes), continuous glucose monitoring can provide a much more valuable source of information than the caloric content or Glycemic index of foods, which are intrinsic properties of food and do not correlate directly with individual metabolic response. Nor does food labeling provide direct, personal feedback about the subject's body, choices or history (change over time). Different people react differently to the same foods, especially under real life conditions of overlapping meals, stress, variable physical activity, medication, hormonal changes and hydration.

The inventive method and system allow for an optimized dietary and behavioral management, wherein food choices can be entirely customized to the individual's needs instead of being dictated by nutritional guidelines, food labeling, popular theories or fad diets. The invention offers a unique way to educate and motivate people in their dietary and exercise goals.

Preferably, the generation of the second data comprises the calculation of an area-under-the-curve (AUC) value. As mentioned above, glycemic response is often expressed or quantified as an "area under the curve," or AUC, being calculated as the difference between measured glucose and fasting glucose. In the context of the present invention the calculated AUC value provides a meaningful but conceptionally simple basis for assessing the effect of a certain event (meal, physical exercise etc.) on the organism of the subject.

If the individual glycemic response budget is given in AUC terms as described above the calculated AUC value may be easily compared to the response budget, i. e. to a reference AUC value corresponding to a recommended total carbohydrate intake during the predetermined time-interval. However, the correlation of AUC to health and nutrition predictions may be based on further quantities such as Glycemic index, Glycemic Load and other quantities that are known from nutritional counseling or medical research.

There are different methods of calculating AUC values. A preferable method termed incremental area under the blood glucose response curve (iAUC) is described in US 2005/0244910 A1 (T. M. S. Wolever et al.): The iAUC describes the area under the blood glucose response curve and above the starting (baseline) concentration, ignoring any area beneath the baseline. Therefore, as long as the offset of the measured glucose values with respect to the starting concentration is known, for calculating the iAUC a relative measurement suffices, it is not necessary to know the absolute value of the starting concentration and a calibrated measurement is not required. As mentioned above, the area under the curve is proportional to the Glycemic Load. Therefore, by calculating the AUC value a direct link to well known weight management methods is created. However, in contrast to usual methods the user does not have to rely on food labeling but he or she gets feedback corresponding to his or her actual metabolic response.

The method may further comprise the step of providing suggestions regarding how to achieve personal metabolic goals. These suggestions may be based on the correlation of the second data with the individual glycemic response budget and/or other quantities, e. g. obtained by further statistical analyses of the glycemic response curves. The suggestions may relate to the choice of foods, portion sizes, times of meals, intensity of physical exercise and may include alerts if certain unfavorable situations (adverse food choices or meal times etc.) are detected by the measurement device and method as well as a sort of gratification system rewarding positive developments.

The analysis as well as the provision of suggestions as mentioned before may be controlled and effected by supplementary software tools to aid in the real-time and/or retrospective interpretation of the measured glycemic data. For this purpose, the inventive system may connect to a database of nutritional and/or health guidelines, e. g. via a data network such as the internet. Furthermore, it may have the possibility to connect to a web-enabled site for social networking, i. e. related to nutritional topics, weight loss, sports or diabetes.

In the context of the invention, a suitable measuring device is a continuous glucose monitoring device. These are computer-operated devices that can be worn, carried or implanted in or on the body for the purpose of continuous glucose measurements. Continuous glucose monitoring (CGM) devices as such are known from the field of diabetes management (they are available e. g. from DexCom, Medtronic Minimed, Abbott etc.). In principle, these known devices meet the demands on a measuring device for the present application for nutritional counseling.

A preferred embodiment of the invention uses an implantable glucose sensor. Such a sensor is preferably coupled to the analysis device by a wireless link. Corresponding sensors are in development, e. g. by Sensors for Medicine and Science, Inc. (SMSI).

Other embodiments comprise a continuous glucose monitoring patch, to be worn on the body of the subject. Such a patch comprises a needle-type, electrochemical glucose sensor which is injected into the subcutaneous tissue. Glucose monitoring patches are very compact and lightweight, reliable and easy to use. They are commercially available, e. g. from the firm DexCom.

Alternatively, a non-invasive glucose monitoring device is employed. Furthermore, in principle most of the aspects of the invention may also be implemented in cases where the glucose level of the subject is measured at a sufficient rate by usual spot blood glucose measurements, e. g. by using traditional strip glucose meters.

In one embodiment of the invention, the measuring device (especially a CGM patch) comprises a storage for temporarily storing the first data. The first data is accumulated in the storage and off-loaded to the analysis device at a later time, in particular after the measuring device has been removed from the body of the subject. The measuring device and the analysis device comprise corresponding transmission components, such as plugs and jacks, chip readers or even wireless interfaces. It is not necessary that the data is directly transmitted from the measuring device to the analysis device but the system may be designed in such a way that the transmission of the data involves off-loading to a first device (e. g. a cellular phone, a PDA or a personal computer) and further transmission to the analysis device (e. g. via the internet or cellular data services). In this embodiment of the invention the measuring device constitutes a compact, lightweight stand-alone recording device for glucose data and allows for retrospective analysis as soon as the accumulated data has been transferred to the analysis device (which may be e. g. a personal computer, a specific web site, a dedicated device for analyzing glucose data or any suitable consumer electronic device). Instead of the patch, an implantable glucose sensor or a non-invasive metabolic (glucose) monitoring device having the described functionality may be employed.

Alternatively, in other embodiments the measured data is continuously submitted to the analysis device. Note that even in this case it may be advantageous to have a storage in the measuring device, especially for buffering the measured data until it has been successfully transmitted.

Preferably, the first data is transmitted to the analysis device via a wireless communication link. For that purpose, the measuring device and the analysis device comprise corresponding wireless transmission components. This allows for hassle-free and automatized communication between the measuring and the analysis devices.

Especially in the case of a wireless link it is preferred that a transmission of first data to the analysis device is activated depending on a value of the measured glucose level. A corresponding method is disclosed by EP 1 688 085 A1 (Disetronic Licensing AG). In the context of the current invention it only makes sense to establish a data connection when there is actual data to transfer from the measuring device to the analysis device, e. g. after a glucose measurement has been performed. Thereby, energy consumption of the devices may be optimized, especially in cases where a wireless link is employed. Furthermore, it is possible not to transmit every measurement to the analysis device, especially in cases where the measured value remains more or less constant or where the measuring rate of the measuring device is higher than required. For these cases it is advantageous to provide the measuring device with storage and computation means that allow for storing and comparing measured values.

Alternatively, the link between measuring and analyzing device is permanent (e. g. if a cable connection is provided or if the two devices are integrated into one unit) or the data is periodically off-loaded as described above.

In a preferred embodiment the analysis device is comprised by a handheld device which is linkable to the measuring device. Furthermore, the handheld device advantageously comprises the analysis device as well as the output device, whereas the second data is displayed on a graphical display of the handheld device. Today's consumer electronic devices such as PDAs, cellular phones, portable digital music players etc. are capable of perform even rather complex analyses and many of them feature high resolution graphical displays. Most of these devices offer a means of wireless communication with other devices (such as Bluetooth, WLAN, IrDA etc.) These devices are easy to use, accepted by the user and inexpensive. All this makes them very appropriate for the present purpose.

Alternatively and/or additionally, a personal computer or a dedicated analysis device is employed. Furthermore, it is possible to employ combined devices comprising the measuring device, the analysis device as well as the output device, e. g. in the form of a "glucose watch".

The step of providing feedback on the output device may involve user interaction in order to control interpretation, i. e. processing and output, of the second data. For this purpose, the analysis and/or output device comprises a user input device and is designed and programmed in such a way that the user may control visualization and interpretation of the second data by using the input device.

Different user interfaces and visualizations may be employed in the context of the present invention. A particularly favorable method for processing and displaying the second data is disclosed by the European patent application No. 06 405 457.0 (F. Hoffmann-La Roche AG) of 31 October 2006. Despite the fact that the disclosed method is designed to be a visual, interactive tool for CGM data for use by people with diabetes, most of the disclosed methods may also be applied to the use with non-diabetic subjects. It involves storing a time segment of the sequence of measured glucose values and simultaneously graphically displaying a plurality of the values of the segment on a user interface display. The segments are assigned to relevant events (such as meals) and the system provides for a database for building-up a library of glucose sequences associated to different events. In particular, the user is able to record and store personally meaningful data intervals. In the context of the present invention, these intervals can be used to calculate individual glycemic response (AUC) for a single food item, meal, period of time (such as a day or week) or athletic event. Personal glycemic response can also be used to evaluate the subject's performance with respect to dietary goals; and may also be used to compare similar events to gauge metabolic variability.

Possible other methods for visual data comparison and analysis are described in WO 2005/087091 A2 (e-san Limited); but whereas this patent describes the application to chronic disease management, in the context of the present invention similar principles for comparing the current metabolic state against historic metabolic states may be applied for use by non-diabetic subjects. Particularly, WO 2005/087091 A2 teaches to simultaneously displaying values of a parameter that are representative of a patient-specific model of normality for that parameter as well as values that are representative of the current condition of the user. In summary, one of the strengths of graphical display lies in the simple comparison of the current response against past responses to a similar event or to a response that is considered to be an ideal response to a certain event.

As an alternative to graphical display of the feedback corresponding to the result of the comparison or additionally to it, it is also possible to display numbers, in particular numbers that have a close connection to values that are relevant for weight management or nutritional counseling, such as percentage of the individual's daily glycemic response budget. Furthermore, instead of displaying the feedback or additionally to it, the output may be auditory, by means of a loudspeaker or ear phones.

In any way, it is preferable that measured glucose data may be saved, recalled and annotated, either in real-time or retrospectively. This allows for building up a library of reference events which e. g. facilitates comparing the current glycemic response with the response on earlier occasions or comparing the achieved results with the individual goals. This also allows for personal notation, i. e. for keeping a personal glucose, sports and nutrition diary.

In order to yield meaningful second data it is necessary that the values measured by the measuring device directly and unambiguously relate to the glucose level of the qualified subject or to an offset of the glucose level with respect to fasting glucose level, respectively. However, in order to achieve that, due to sensor drift and other sources of error, it is necessary to regularly calibrate the sensor. Conventionally, this is done by performing spot measurements of the blood glucose level, e. g. by employing a conventional strip glucose meter. However, these measurements ask for an additional effort of the user.

Therefore, preferably, the method comprises a step of self-calibration for the measuring device, comprising the step of establishing the reference value for the fasting glucose level of the subject, to be used as a reference for generating the second data. Thereby, calibration with independent blood glucose measurements may be minimized or avoided.

Advantageously, the step of self-calibration is automatically and regularly effected during periods without ingestion of foods and glucose-affecting beverages by the subject, in particular regularly overnight. This assures that the calculated fasting glucose level is continuously calibrated against the measured fasting glucose level. It is possible to perform the step of self-calibration based on a time signal (e. g. every 24 hours, regularly at 05:00 in the morning) and/or it may be performed ex post, after the device has established a long enough period without glucose-relevant ingestion of foods and beverages, based on the performed glucose measurements (cf. EP 1 728 468 A1, F. Hoffmann-La Roche AG, Roche Diagnostics GmbH).

In a particularly advantageous embodiment, the step of self-calibration comprises the following substeps
a) monitoring the subject's glucose level during a minimum of six, in particular during a minimum of eight, consecutive hours without ingestion of foods and beverages;
b) determining when glucose has stabilized at a fasting level
c) averaging a signal corresponding to a measured glucose concentration during an interval of greatest signal stability in order to determine the reference data corresponding to the fasting glucose level.

The length of the interval may be predetermined, e. g. 2 hours. It is chosen from the whole measuring period by using statistical methods such as running averages or a standard deviation of the measured glucose values or of the glucose rate-of-change, respectively. One possible method to determine signal stability is described in EP 1 728 468 A1 (F. Hoffmann-La Roche AG, Roche Diagnostics GmbH).

What is determined from these steps is the value of the sensor signal corresponding to the user's fasting glucose level. This value is later used as a reference data for converting the measured values of the sensor signal when monitoring the subject's glucose level and when calculating the second data, e. g. when prandial glucose is measured against fasting glucose for the purpose of calculating iAUC. It is important to note that in the context of this invention it is generally not necessary to know the absolute value of the subject's fasting glucose level (say the actual value in mg/dl) but only the difference of the actual glucose level and the fasting glucose level. This is in contrast to diabetes management where usually the absolute value has to be determined.

If it is noticed during the process of self-calibration that the glucose level rises due to the user having a meal or drink and/or that a stable fasting glucose level is not reached, the value of the previous self-calibration step will be used until a later self-calibration is successful.

Furthermore, the inventive method advantageously includes the step of correcting a raw signal corresponding to the measured glucose level against drift, signal instability or system error, especially if regular system calibration by blood glucose measurements is to be eliminated. For this purpose, the measuring device and/or the analysis device comprises a computer and/or an analog electronic circuit for filtering noise and/or for correcting a raw signal corresponding to the measured glucose level against drift, signal instability or system error. In principle, corresponding methods are known, see US 2005/272985 A1, EP 1 518 495 A1, US 2005/240356 A1, US 2006/052679 A1 and especially US appl. No. 11/680,963 of 01 March 2007 (all of Roche Diagnostics). According to the document cited last the method of correcting the signal includes the steps of applying a time-varying input signal to at least one of the one electrode of the sensor, monitoring a time-varying output signal produced by the sensor in response to application of the time-varying input signal, determining a complex impedance of the sensor based on the time-varying input signal and output signals, and determining from the complex impedance information relating to operation of the sensor. This information is subsequently used for determining the actual value measured by the sensor.

Other advantageous embodiments and combinations of features come out from the detailed description below and the totality of the claims.

### Brief description of the drawings

The drawings used to explain the embodiments show:
- Fig. 1A: A schematic representation of an inventive system for monitoring individual metabolic response, involving monitoring of glycemic response in a qualified subject;
- Fig. 1 B: a schematic representation of a continuous glucose measuring patch for another inventive system for monitoring individual metabolic response;
- Fig. 2: a schematic representation of a glucose progression measured with a measuring device of the inventive system;
- Fig. 3A, B: flowcharts representing an inventive method for monitoring individual metabolic response in real-time and involving data accumulation and off-loading;
- Fig. 4A-C: an illustration of the calculation of the area under the curve, the accumulated area under the curve as well as its comparison to a daily glycemic response budget;

- Fig. 5: the graphical user interface for displaying the glucose progression, glycemic budget and a suggestion for achieving personal metabolic goals;
- Fig. 6: the archive menu of the graphical user interface, displayed on computing and display equipment;
- Fig.7: the graphical user interface for choosing a time interval from prerecorded data;
- Fig. 8: the directory structure for storing and retrieving archived responses into and from the database, respectively;
- Fig. 9: the thumbnail representation of a response within a given directory;
- Fig. 10: the detailed view of an archived response; and
- Fig. 11: the graphical user interface for comparing a current glycemic response to an archived response.

In the figures, the same components are given the same reference symbols.

### Preferred embodiments

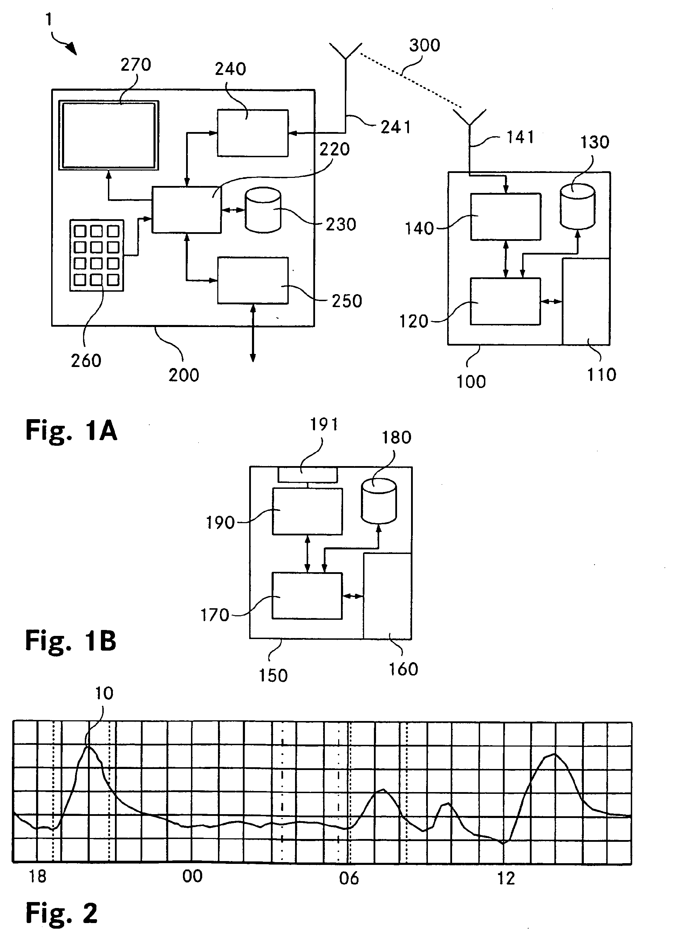

The Figure 1A is a schematic representation of an inventive system for monitoring individual metabolic response, involving monitoring of glycemic response in a qualified subject. The system 1 comprises a glucose measuring device 100 as well as a computing and display equipment 200. The two devices are linked by a wireless RF connection 300.

In the given example, the glucose measuring device 100 is to be placed on a human body and continuously measures glucose values in interstitial fluid by means of an electrochemical (alternatively: photometric) glucose sensor 110. The measuring device 100 further comprises an extra corporal part including a central processing unit (CPU) 120, a storage 130 connected to the CPU 120 and an interface unit 140. The CPU 120 controls the sensor 110 and periodically stores the blood glucose value that is actually measured in storage 130. Suitable frequencies for taking measurements are from 4 (i. e. a measurement every fifteen minutes) to 60 (i. e. a measurement every minute) measurements an hour. Periodically, the measurements stored in storage 130 are transmitted to the glucose measuring device 100 by means of the wireless RF connection 300. For this purpose, the data to be transmitted is first transmitted to the interface unit 140 by the CPU 120. The interface unit 140 pre-processes the data to be sent; this pre-processing step may include encryption of the data. Furthermore, the interface unit 140 includes a transceiver linked to an antenna 141. In one embodiment of the invention the glucose measuring device 100 comprises an arm cuff that inductively powers and communicates with a glucose sensor that is implanted in the user's arm. The arm cuff features the components of the extra corporal part and communicates with a handheld device as described in the following.

The RF signal is received by an antenna 241 of the computing and display equipment 200. This equipment further comprises an interface unit 240 connected to the antenna, including a transceiver as well as a processing stage for processing the received signals as well as signals to be transmitted (see below). The equipment 200 is controlled by a central processing unit (CPU) 220 which is connected to a storage 230, a further interface unit 250, a user input device 260 and a display 270. Controlled by the CPU 220 the received measurements may be stored in storage 230 as well as displayed on the display 270. By means of the further interface unit 250 the computing and display equipment 200 may be linked to further electronic devices such as a Personal Computer (PC) of the user or the nutritionist or further data gathering and/or storage devices such as pulsometers, pedometers, electronic scales, blood glucose meters, cellular phones, personal digital assistants (PDA) etc. This allows for automatically obtaining at least part of the meta-data (physical exercise of the user, results of individual blood glucose measurements or weight measurements, etc.) to be stored in the database.

Besides for transmitting measured values from the glucose measuring device 100 to the computing and display equipment 200 the wireless RF connection 300 also serves for transmitting control data from the equipment 200 to the measuring device 100, e. g. for changing the measurement frequency or to initiate the transmission of the data stored on the glucose measuring device 100, if the transmission is usually initiated by the equipment 200 (polling mode).

The computing and display equipment 200 may be implemented by a personal digital assistant (PDA, including portable music/multimedia players), a personal computer, a cellular or smart phone, an analyte measuring device such as a glucose measuring device, e. g. a hand held glucose meter, more preferably a strip based glucose meter, or combinations thereof. Some of these devices usually comprise most or all of the components described above: as an example, a PDA usually features wireless as well as wire-based connection interfaces (e. g. Bluetooth and USB, respectively), a rather powerful CPU, storage means (e. g. internal Flash storage and replaceable memory cards), user input devices (keys, touchpad, touchscreen etc.) as well as a display (e. g. a high resolution color LCD display). Therefore, in these cases it is sufficient to provide specific software adapted to the actual equipment 200 that provides the desired functionality of the inventive system.

The Figure 1B is a schematic representation of a continuous glucose measuring patch 150 for another inventive system for monitoring individual metabolic response.

The patch 150 is to be placed on a human body and continuously measures glucose values in interstitial fluid by means of a needle-type, electrochemical glucose sensor 160. The patch 150 further comprises a central processing unit (CPU) 170, a storage 180 connected to the CPU 170 and an interface unit 190 connected to a connector 191. The CPU 170 controls the sensor 160 and periodically stores the glucose value that is actually measured in storage 180 such that the measured values are accumulated. Suitable frequencies for taking measurements are from 10 (i. e. a measurement every six minutes) to 600 (i. e. a measurement every 10 seconds) measurements an hour. After the patch has been removed from the body of the user it may be connected to a further device (such as a computing and display equipment as described in connection with Figure 1A, a personal computer or another device) by means of the connector 191. Subsequently, the measurements stored in storage 180 are transmitted to the further device. For this purpose, the data to be transmitted is first transmitted to the interface unit 190 by the CPU 170. The interface unit 190 pre-processes the data to be sent; this pre-processing step may include encryption of the data. The connector 191 can be a jack-type connector providing for a direct electrical connection or it can provide for an inductive or capacitive coupling.

The Figure 2 is a schematic representation of a glucose progression measured with a measuring device of the inventive system, e. g. in units of mg/dl. The displayed progression represents 24 hours, starting and ending at 17:00. From the rising sections of the curve 10 it is clearly visible that the user had meals around 19:00, 06:00, 09:30 and 12:30.

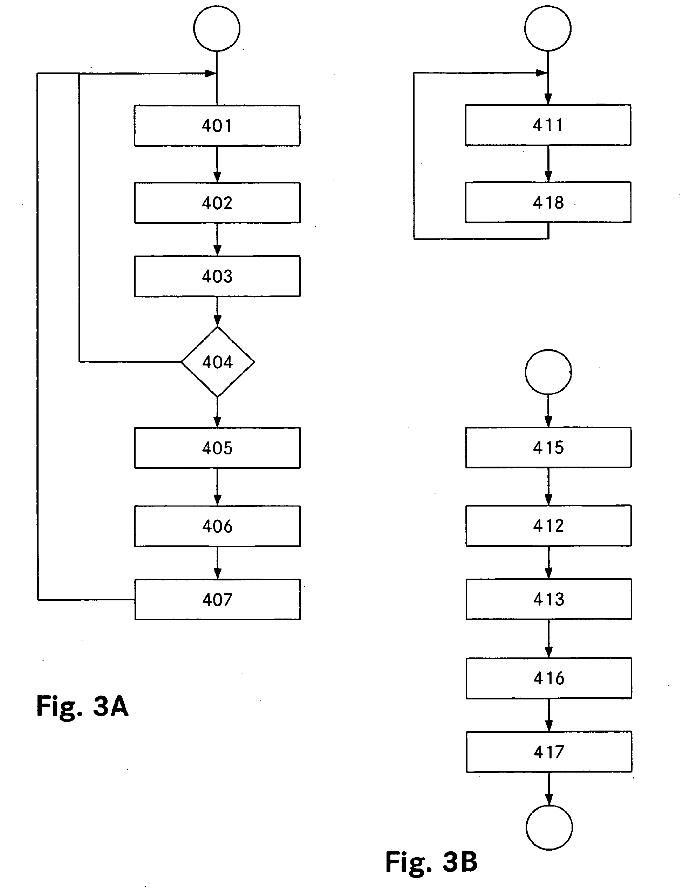

The Figures 3A, 3B are flowcharts representing an inventive method for monitoring individual metabolic response in real-time and involving data accumulation and off-loading, respectively. The Figure 3A represents the real-time process. First of all, a measurement of the glucose level is taken (step 401). The measured signal is subsequently corrected against drift, signal instability and system error (step 402), in particular according to the method as described in US appl. No. 11/680,963 of 01 March 2007 (Roche Diagnostics). The resulting corrected signal is converted to a glucose level value (e. g. in mg/dl) (step 403). In a next step 404 the actually measured glucose level value is compared to a reference value corresponding to the previously measured value. If the difference of the two values does not exceed a certain minimum threshold (such as e. g. 3 mg/dl) no further action is taken and the process continues with taking another measurement (step 401) after a predetermined time.

Only if the minimum threshold is exceeded the actual glucose level value is transmitted to an analysis device (step 405). For the event of very long stable intervals, the process may involve the transmission of the value even if the difference does not exceed the minimum threshold, provided that a certain minimum interval has elapsed since the last transmission (e. g. 1 hour). This ensures that failures of the glucose measuring device inhibiting the transmission of measured values to the analysis device are not mistaken for stable glucose progressions.

In the analysis device the transmitted glucose level value is used to generate data representing the glycemic response of the user (step 406); in particular, the area under the curve (AUC) is calculated and the AUC is compared with an individual glycemic response budget as will be described below. Finally, the updated data is displayed on a display device (step 407). The process (steps 401-407) is repeated in a cycle in order to ensure constant updates of the displayed information. The displaying step 407 may involve user interaction in order to control the display of the data, to update or modify a database of metabolic response data, to control the operation of the device etc. (see below).

The Figure 3B represents the retrospective process. In a continuous glucose measuring patch as described in connection with Figure 1B above, a measurement of the glucose level is taken (step 411). The measurement is subsequently stored in a storage of the patch (step 418). These steps are repeated as long as the patch is placed on the body of the user.

After the patch has been removed from the body the accumulated data stored in the storage is transmitted to an analysis device (step 415). Using further data stored on the storage of the patch the transmitted data is corrected against sensor drift, signal instability and system error (step 412), in particular according to the method as described in US appl. No. 11/680,963 of 01 March 2007 (Roche Diagnostics). The resulting corrected signal is converted to a glucose level value (e. g. in mg/dl) (step 413). This value is used to generate data representing the glycemic response of the user (step 416); in particular, the area under the curve (AUC) is calculated and the AUC is compared with an individual glycemic response budget as will be described below. Finally, the data corresponding to the event is displayed on a display device (step 417). Again this last step may involve user interaction.

The patch may be placed on the body again to collect further data.

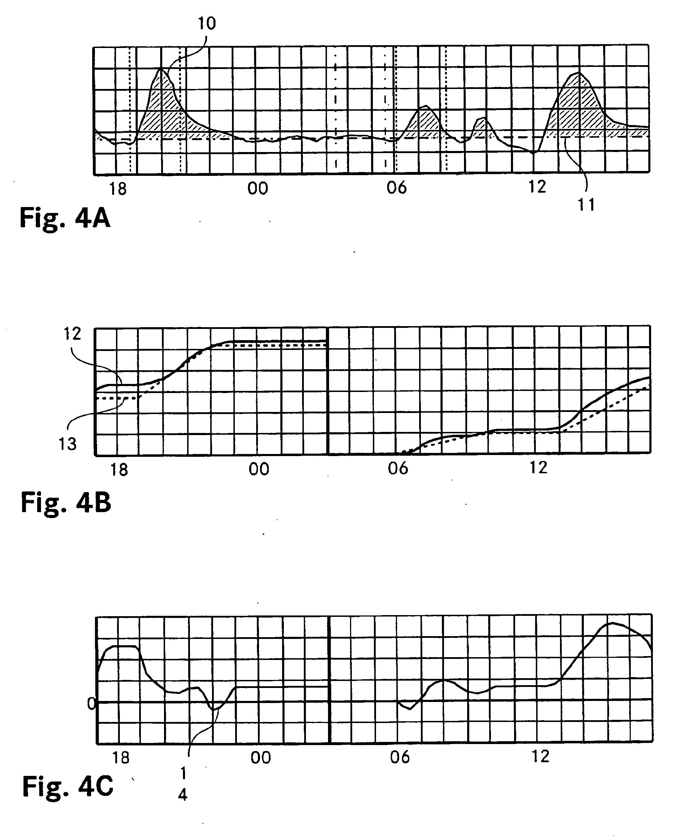

The Figures 4A-C illustrate the calculation of the area under the curve (AUC), the accumulated area under the curve as well as its comparison to a daily glycemic response budget (GRB). In Figure 4A the glucose progression 10 of Figure 2 is displayed again. Based on the measured glucose progression 10 the fasting glucose level is regularly and automatically determined, if possible the fasting level is updated once each 24 hours. For this purpose, a period is chosen that corresponds to a minimum of six consecutive hours without ingestion of foods and (glucose-relevant) beverages, usually during the night, when the user is asleep. In the given example, the glucose level does not significantly rise between about 20:00 and 06:00, therefore it may be assumed that these ten hours are a suitable period. Next, it is determined at which point during this interval glucose has stabilized at a fasting level. This can be done by known techniques for signal analysis, e. g. by monitoring the rate-of-change of the glucose level and by establishing stability if the rate falls below a certain threshold and stays below that threshold for a given minimum time. In the current example, stability is reached at around midnight (00:00).

Next, the measured glucose concentration is averaged during an interval of greatest signal stability. This step may involve calculating averages as well as associated statistical information (such as standard deviations) corresponding to a plurality of intervals during the stable interval from 00:00-06:00. The average having the lowest statistical error or uncertainty is chosen to represent the user's fasting glucose level. This level is displayed as a baseline 11 in Figure 4A.

In order to calculate the area under the curve (AUC), more precisely the incremental area under the curve (iAUC), the difference of the measured glucose value and the fasting glucose level is integrated for all intervals in which the measured glucose value is larger than the fasting glucose level. This is equivalent with determining the area between the baseline 11 and the glucose progression 10, ignoring intervals where the glucose progression 10 falls below the baseline 11. Several methods for calculating the iAUC from a measured glucose progression exist and are applicable in the context of the invention. A suitable method is disclosed e. g. by US 2005/0244910 A1 (T. M. S. Wolever et al.).

The Figure 4B shows the accumulated incremental area under the curve 12 (in units of mg·min/dl), being the sum of iAUC during the present day, assuming that by default the daily counter is set back to zero at 03:00. Due to the fact that there are no "negative areas" under the curve the accumulated iAUC 12 is monotonically rising until the counter is set back to zero.

Further indicated in the Figure 4B is a predetermined target curve 13 corresponding to the proposed progression of the glycemic response matching the predetermined individual glycemic response budget. This target curve 13 starts at zero at the time of setting back the counter (03:00) and ends at the predetermined daily iAUC budget 24 hours later. In order to take into account the daily routine of the user, the target curve 13 is not linear but consists of linearly rising sections during and after expected meal times as well as constant sections in between the rising sections. Within the context of the invention, the progression of the target curve 13 may be further refined, e. g. based on an average of a plurality of recorded daily glucose progressions of the user. It is even possible to dynamically update the target curve 13 during the day, e. g. to adapt the curve 13 if it is detected that a meal starts earlier or later than expected. In any way, a comparison of the accumulated iAUC 12 with the target curve 13 should provide the user with a reliable feedback if he or she is still "on track" for reaching his or her personal metabolic goals.

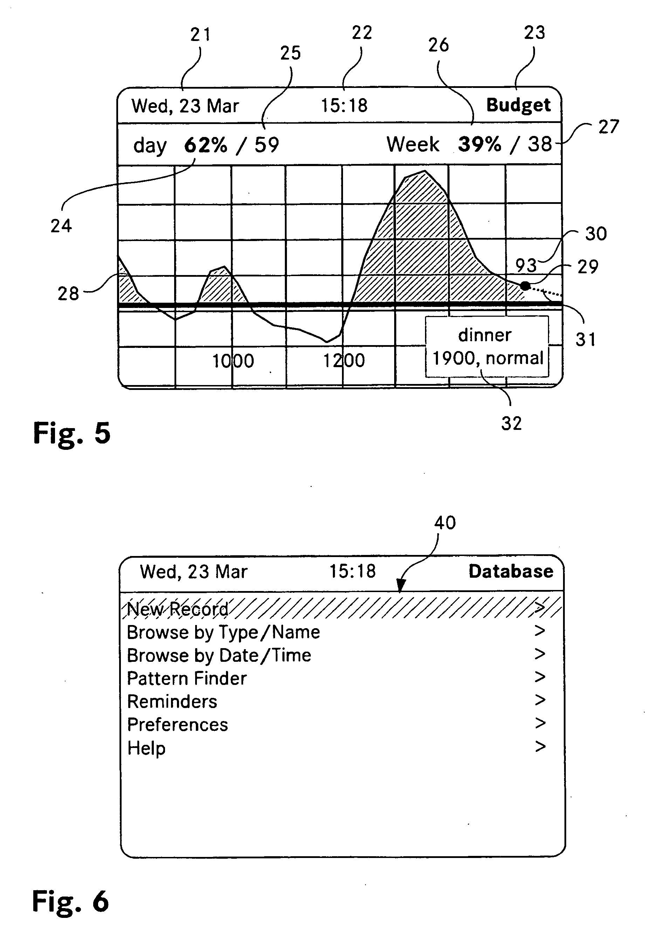

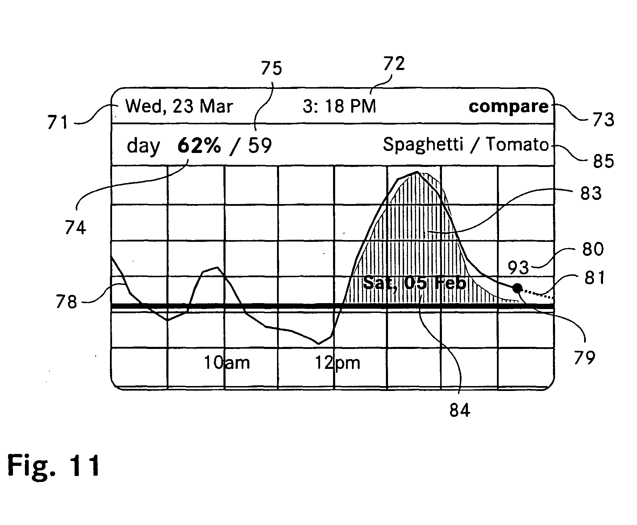

In Figure 4C the difference 14 between the accumulated iAUC 12 and the target curve 13 is displayed. A positive difference suggests that the user should reduce his or her caloric intake and/or increase his or her physical activities in order to reach the predetermined personal metabolic goals. In the context of the invention it is possible to merely display this difference or to supplement the displayed items as described below (Figures 5, 11) with this additional quantity. Alternatively or additionally, the difference may be displayed as a relative quantity (e. g. percentage of proposed glycemic response budget) or in the form of a gauge, e. g. having a "green", "yellow" and "red" sector standing for "good", "average" and "poor" glucose control.

The Figure 5 displays the graphical user interface for displaying the glucose progression, glycemic budget and a suggestion for achieving personal metabolic goals. The graphical user interface (GUI) may be displayed on a computing and display equipment. In the displayed mode, the following information is provided:
a) the current date 21;
b) the current time 22;
c) an indication 23 of the current display mode ("Budget");
d) the use 24 of the glycemic response budget for the current day, compared to a target value 25;
e) the use 26 of the glycemic response budget for the current week, compared to a target value 27;
f) the glycemic response curve 28 of the last 7-8 hours as well as a mark 29 representing the most recently measured glucose level, including the value 30 of this level (in mg/dl);
g) a prediction 31 of the future progression of the glucose level; and
h) a suggestion 32 regarding how to achieve the personal metabolic goals of the user.

By comparing the use of the daily or weekly response budget to the target values the user may readily recognize whether he or she is "on track", whether there is some leeway for choosing his or her diet or whether action is required for reducing the intake of food and/or for increasing physical activity if the personal goals are still to be reached. In the current example, on Wednesday afternoon, at 15:18 the user has used 62% of the daily glycemic response budget and 39% of the weekly glycemic response budget. The target values at the given point in time are 59% and 38%, respectively. This information is obtained from the comparison of the accumulated incremental area under the curve with the target curve corresponding to the individual glycemic response budget as explained above, in connection with Figure 4B. The information displayed in Figure 5 means that the user is slightly above the given budget that corresponds to the metabolic goals of the user, however he or she is still close to the optimum value. Therefore, as no immediate response is necessary, the suggestion 32 is to have a "normal" dinner at 19:00. If the budget is further transgressed the device will suggest to reduce caloric intake and/or to increase physical activity in order to reach the weekly target glycemic response budget.

The prediction 31 of the future progression of the glucose level may be calculated according to known methods. In the simplest case the current steepness of the glycemic response curve is determined and the future progression is assumed to follow a linear curve having the calculated steepness. If a more precise prediction is required or if the time during which a prediction is needed is to be increased above about one hour more elaborate methods may be employed, e. g. methods that include the calculation of higher derivatives and/or the fitting of curves to the measured data points.

It is further possible to deduce a prediction for approximate weight gain or weight loss based on the daily/weekly or monthly glycemic response budget and to display this prediction along with or instead of the other information as described above. In order to obtain reliable predictions it is useful to have personal information correlating the measured glycemic response with actual weight gain or loss measured by means of usual scales. The weight measurements may be supplied to the analysis device by the user, using the user input device, or in the case of electronic scales they may be directly transmitted to the corresponding interface of the analysis device. Comparing the measured glycemic responses to the weight measurements of the same time period a personal correlation profile may be generated which may subsequently be used to deduce predictions of an approximate weight gain or loss depending on the measured glycemic response.

The Figure 6 shows the database menu of the graphical user interface, displayed on a computing and display equipment. In the given example, the graphical user interface resembles the Apple-lpod interface. Correspondingly, choosing from menu options or adjusting parameters can be effected using a clickwheel. However, other input means such as a touch screen, a touch pad or conventional keys and/or other user interfaces (such as user interfaces provided by the operating systems Microsoft Windows, Linux, MacOS, Symbian, or others) are appropriate as well. The corresponding menu structure may be realized on other equipment such as PDAs, mobile/smart phones etc.

The menu 40 shown in Figure 6 allows for choosing from the following options:
a) New Record
b) Browse by Type/Name
c) Browse by Date/Time
d) Pattern Finder
e) Reminders
f) Preferences
g) Help

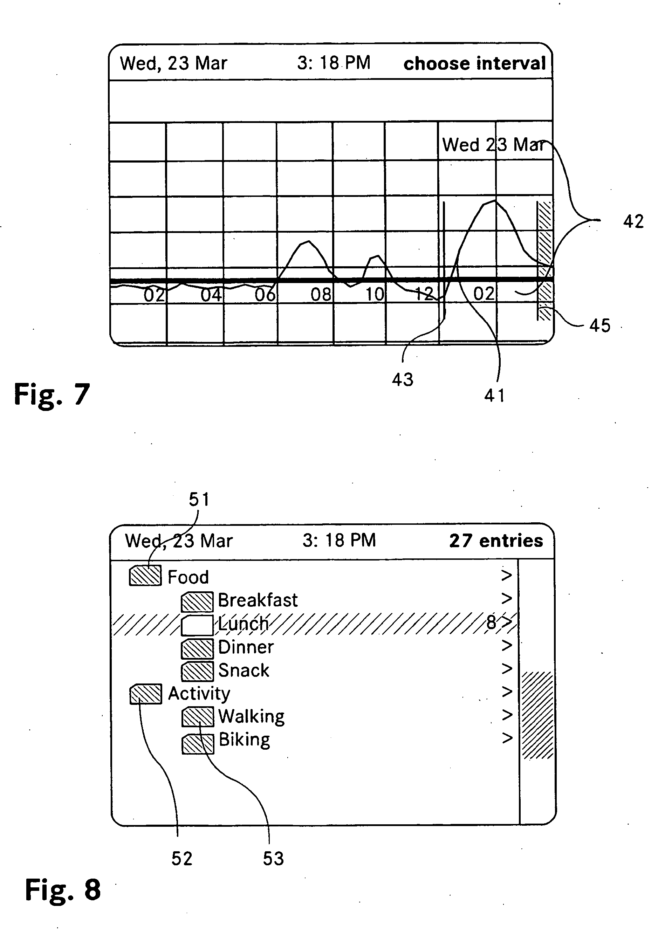

A new record may be generated by choosing option a). The Figure 7 shows an option for defining the time interval in which the measured glucose values will contribute to the recorded response.

In the course of generating a new record the user will be prompted for a name. The name should be a short but meaningful description of the corresponding event (e. g. "Pizza" or even "Pizza for lunch") and will serve as a kind of "file name". It is one of the prime identifiers of the event (besides further meta-data such as time and date, food portion size etc., see below). Further meta-data may be gathered by querying the user or from external devices such as pulsometers, pedometers, cellular phones, personal digital assistants (PDA) etc. or personal computers and automatically stored in the database.

By choosing options b) and c) from the database menu as shown in Figure 6 records stored earlier in the database may be retrieved, employing different criteria. The shapes may be browsed by type and name (see Fig. 8, comment below) or by date and time.

Option d) allows for finding patterns, i. e. earlier records that match a certain shape. Option e) allows for defining, editing and deleting reminders. These reminders may be triggered by a number of events: the lapse of a certain time period (count down), a certain point in time, reaching a certain glucose level or predefined events regarding the glucose level or the used response budget (passing of a maximum/minimum, exceed a bG gradient etc.) The reminders may have a mere warning function or they may be displayed in combination with a prompt that invites the user to provide information or that proposes certain actions (as starting to record measurements for generating a new shape). By choosing option f) certain user preferences (display brightness and contrast, colors, screen saver, graph options, measuring options etc.) may be edited. Finally, option g) displays a help menu, providing access to various documentation about using the software.

Each record stored in the database corresponds to a certain time interval. These intervals may be automatically assigned by the inventive device, or they may be defined by the user. The interval may be defined beforehand, during the interval or even after it has ended. The Figure 7 shows a suitable graphical interface for defining an interval that has already ended. The measurements received from the measuring device are continuously stored in the storage of the computing and display equipment, in such a way that the progression of the glucose level during a certain time span (e. g. 16 hours) before the actual time is always available. The progression of the glucose level is displayed as a curve 41, together with time and date information 42 ("Wed 23 Mar", "14 16 18 ... 02"). By shifting a start bar 43 as well as and end bar 45 the time interval in which the measured glucose values shall contribute to a new record may be defined by the user. In order to obtain standardized shapes consisting of 1-hour segments the chosen interval is extended to the next full hour. The maximum recording time is limited to 6 hours, in order to ensure adherence to the event context.

After the user has defined the time interval a new record is automatically generated. Subsequently, the user may amend the new instance with further information, such as a title and a description. Finally, the record is stored in an event type directory (see above and Figure 8).

The time span during which the progression of the physiological parameter is still available and accessible by the user is deliberately chosen to be limited to about 1-2 days, in order to ensure that the information supplied by the user relating to the time span and the corresponding event (food intake, physical activity etc.) is as correct as possible. In principle, it is possible to store information on the device that relates to longer time spans, however this information should not be eligible for generating new instances and records. It could however be valuable for the patient's HCP.

Figure 8 shows the directory structure for storing and retrieving records into and from the database, respectively, where the shapes are hierarchically grouped by event type. On a first (top) level the events are divided into two groups ("Food", "Activity") containing events 51 that are related to ingestion and events 52 that are related to physical activity. On a second level, the events are further classified into specified event types 53 that relate to specific contexts (such as in the given example breakfast, lunch, dinner, snack for ingestion events, as well as walking, biking for physical activity events). The user is free to create further, custom event types and/or groups.

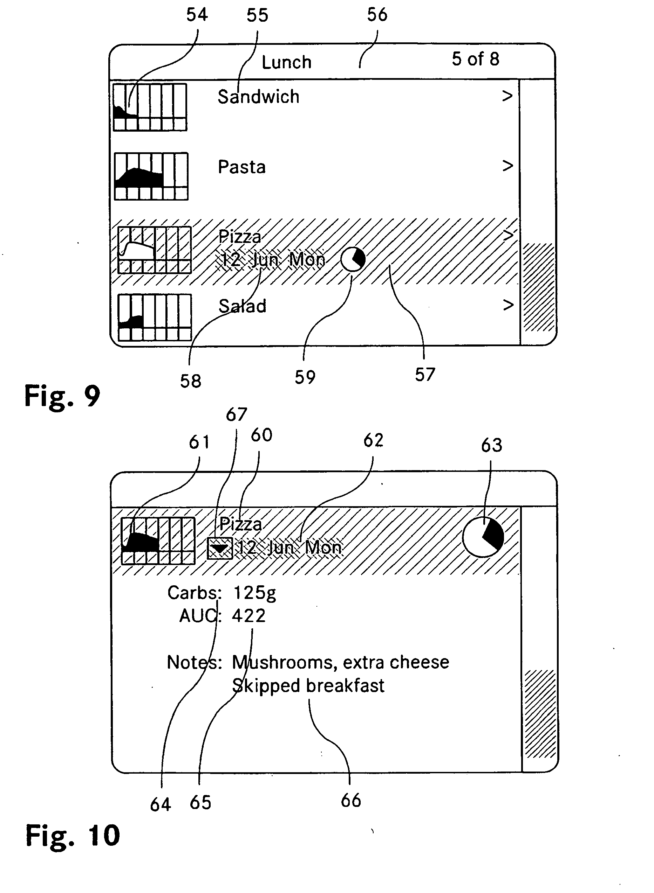

Once a given event type directory is chosen, the contained records ("Sandwich, Pasta, Pizza, Salad") are displayed, as is shown in Figure 9. This includes the display of a thumbnail representation 54 of every event within the given directory as well as of the names 55 assigned to all the displayed records. The title bar 56 shows the name of the directory that corresponds to the name of the event type ("Lunch"). For the record 57 that is currently highlighted additionally the date and weekday 58 ("12 Jun Mon") as well as the time and interval 59 of the latest recorded incident are displayed. The time and interval information 59 is given as a marked segment of a clock face. This allows for quickly identifying the relevant information.

Figure 10 shows the detailed view of a record that appears once it has been chosen from the event directory displayed in Figure 9. The detailed view shows the information discussed above in relation with Figure 9, i. e. the name 60 of the record ("Pizza") as well as the shape 61, date/weekday 62 ("12 Jun Mon") and time/interval 63 of the incident that has been most recently recorded. In a lower part of the display additional information relating to the displayed incident is provided such as the amount of carbohydrates 64 of the meal ("Carbs 125 g") provided by the user, the elicited glycemic response 65 ("AUC: 422", in units of mg·min/dl) as well as notes 66 that are provided by the user (e. g. further information concerning the ingredients of the meal or concerning special circumstances, in the given example "Notes: Mushrooms, extra cheese, Skipped Breakfast"). The notes may be provided or amended at any time. However, in order to ensure accurate information the user will be prompted for the information immediately after creation of the record.

By default the most recently recorded incident is displayed. However, previous incidents of the same event may be easily accessed by means of a pulldown menu 67.

The Figure 11 displays the graphical user interface for a comparison between the present glucose progression and an earlier glucose progression stored as a record in the database. The user interface is similar to the one shown in Figure 5, displaying:
a) the current date 71;
b) the current time 72;
c) an indication 73 of the current display mode ("compare");
d) the use 74 of the glycemic response budget for the current day, compared to a target value 75;
e) the glycemic response curve 78 of the last 7-8 hours as well as a mark 79 representing the most recently measured glucose level, including the value 80 of this level (in mg/dl);
f) a prediction 81 of the future progression of the glucose level.

Furthermore, a shape 83 representing the glucose progression of a stored record is superposed to the glycemic response curve 78. The shape 83 is denoted by its date 84 ("Sat, 05 Feb") as well as by the title 85 ("Spaghetti/Tomato") of the record.

By comparing the present glucose progression to a stored record belonging to a similar event (in particular to a similar meal) the user may readily recognize if the response to the relevant event has changed, e. g. because the state of health of the user has changed or if the user has worked out during the time preceding or following the lunch.

Further useful features of a graphical user interface which is suitable for the current invention are described in EP 06 405 457.0 (filed 31 October 2006, F. Hoffmann-La Roche AG).

The invention is not restricted to the embodiments described above. Other sensor devices or analysis devices may be used. It is possible to vary the measurement frequency and the mode and means of transmission of the data from the sensor device to the analysis device. In principle, the measurements may be normalized, compensated and/or error corrected by employing usual methods known from the prior art. Furthermore, the analysis of the series of measurements and the output of the measured and/or determined quantities may as well happen in modified form.

In summary, it is to be noted that the invention provides for a method for monitoring individual metabolic response, involving monitoring of glycemic response in a qualified subject, that is comfortable for the user and that provides a personalized and specific feedback supporting the user's dietary management.

## Claims

1. A method for monitoring individual metabolic response and for generating nutritional feedback, involving monitoring of glycemic response in a qualified subject, comprising the steps of:
a) consecutively performing a plurality of measurements of a glucose level in the qualified subject by a measuring device (100; 150);
b) in the measuring device (100; 150) generating first data corresponding to the measured glucose level;
c) transmitting the first data to an analysis device (200) and providing feedback on an output device (270);
**characterized in that**:
d) in the analysis device (200) second data is generated representing a glycemic response of the subject involving comparing a time-series of glucose measurements represented by the first data with a reference value for the fasting glucose level of the subject;
e) the second data is compared with a predetermined individual glycemic response budget for the qualified subject, the individual glycemic response budget representing a total amount of individual glycemic response allowable for a certain time period corresponding to a recommended total carbohydrate intake during this time period; and
f) feedback is provided corresponding to a result of the comparison of the second data with the predetermined individual glycemic response budget on the output device (270).

2. The method as recited in claim 1, **characterized in that** the step d) comprises the calculation of an area-under-the-curve (AUC) value, in particular of an incremental area-under-the-curve (iAUC) value.

3. The method as recited in claim 2, **characterized in that** the step e) comprises the comparison of the calculated area-under-the-curve (AUC) value corresponding to a predetermined time-interval, in particular 24 hours or a week, to a reference AUC value corresponding to a recommended total carbohydrate intake during the predetermined time-interval.

4. The method as recited in any of claims 1 to 3, **characterized in that** it further comprises the step of providing suggestions regarding how to achieve personal metabolic goals.

5. The method as recited in any of claims 1 to 4, **characterized in that** the measuring device (100; 150) is a continuous glucose monitoring device, in particular an implantable glucose sensor.

6. The method as recited in any of claims 1 to 5, **characterized in that** a transmission of the first data to the analysis device (200) is activated depending on a value of the measured glucose level.

7. The method as recited in any of claims 1 to 6, **characterized in that** the step of providing feedback on the output device (270) involves user interaction in order to control interpretation of the second data.

8. The method as recited in any of claims 1 to 7, comprising a step of self-calibration for the measuring device (100; 150), comprising the step of establishing the reference value for the fasting glucose level of the subject.

9. The method as recited in claim 8, **characterized in that** the step of self-calibration is automatically and regularly effected during periods without ingestion of foods and glucose-affecting beverages by the subject, in particular regularly overnight.

10. The method as recited in claim 8 or 9, **characterized in that** the step of self-calibration comprises the following substeps:
a) monitoring the subject's glucose level during a minimum of six, in particular during a minimum of eight, consecutive hours without ingestion of foods and beverages;
b) determining when glucose has stabilized at a fasting level; and
c) averaging a signal corresponding to a measured glucose concentration during an interval of greatest signal stability in order to determine the reference data corresponding to the fasting glucose level.

11. The method as recited in any of claims 1 to 10, comprising a step of correcting a raw signal corresponding to the measured glucose level against drift, signal instability or system error.

12. A glucose monitoring system for monitoring glycemic response in a qualified subject and for generating nutritional feedback, comprising
a) a measuring device (100; 150) comprising a sensor for consecutively performing a plurality of measurements of a glucose level in the qualified subject and comprising a data generator for generating first data corresponding to the measured glucose level;
b) an analysis device (200) for analysing the first data; and
c) an output device (270) for providing feedback;
**characterized in that**:
d) the analysis device (200) comprises a computer configured to generate second data from the first data, the second data representing a glycemic response of the subject and the generation of the second data involving comparing a time-series of glucose measurements represented by the first data with a reference value for the fasting glucose level of the subject, and to compare the second data with a predetermined individual glycemic response budget for the qualified subject, the individual glycemic response budget representing a total amount of individual glycemic response allowable for a certain time period corresponding to a recommended total carbohydrate intake during this time period; and
e) the output device (270) is computer-controlled and arranged to provide feedback corresponding to a result of the comparison of the second data with the predetermined individual glycemic response budget.

13. The system as recited in claim 12, **characterized in that** the measuring device (150) is an implantable glucose sensor.

14. The system as recited in claim 13, **characterized in that** the measuring device (150) comprises a storage (180) for temporarily storing the first data and **in that** the measuring device and the analysis device (200) comprise transmission components (190, 191, 240) for off-loading of the accumulated stored first data to the analysis device (200), in particular after the measuring device (150) has been removed from the body of the subject.

15. The system as recited in any of claims 12 to 14, **characterized in that** the measuring device (100) and the analysis device (200) comprise transmission components (140, 141, 240, 241) for transmitting the first data from the measuring device to the analysis device (200) via a wireless link, further **characterized in that** the analysis device comprises a storage for storing the received first data.

16. The system as recited in any of claims 12 to 15, **characterized by** a handheld device (200) comprising the analysis device and the output device.

17. The system as recited in any of claims 12 to 16, **characterized in that** the analysis device comprises a storage for storing the predetermined individual glycemic response budget for the qualified subject.

18. The system as recited in any of claims 12 to 17, **characterized in that** the measuring device and/or the analysis device comprises a computer and/or an analog electronic circuit for filtering noise and/or for correcting a raw signal corresponding to the measured glucose level against drift, signal instability or system error.

19. The system as recited in any of claims 12 to 18, **characterized in that** the analysis device and/or the output device comprises a user input device (260) and **in that** it is designed and programmed in such a way that a user may control interpretation of the second data by using the input device (260).

20. The system as recited in any of claims 12 to 19, **characterized in that** the analysis device comprises a storage for storing second data as well as user-specified information.

21. Computer program product including program code which when executed on an analysis device carries out the following steps :
a) generating second data representing a glycemic response of a qualified subject from first data, involving comparing a time-series of glucose measurements in a qualified subject, represented by the first data, with a reference value for the fasting glucose level of the subject;
b) comparing the second data with a predetermined individual glycemic response budget for the qualified subject, the individual glycemic response budget representing a total amount of individual glycemic response allowable for a certain time period corresponding to a recommended total carbohydrate intake during this time period; and
c) generating at least one quantity relating to nutritional feedback based on the result of the comparison of the second data with the predetermined individual glycemic response budget.

## Patentansprüche

1. Verfahren zur Überwachung der individuellen Stoffwechselantwort und zur Erzeugung von Ernährungsfeedback, mit Überwachung der glykämischen Antwort in einer geeigneten Person, das folgende Schritte umfasst:
a) Durchführung einer Vielzahl von aufeinanderfolgenden Messungen eines Blutzuckerspiegels bei der geeigneten Person mit einem Messgerät (100; 150);
b) im Messgerät (100; 150) Erzeugung von ersten Daten, die dem gemessenen Blutzuckerspiegel entsprechen;
c) Übertragung der ersten Daten zu einem Analysegerät (200) und Bereitstellung von Feedback auf einer Ausgabevorrichtung (270);
**dadurch gekennzeichnet, dass**:
d) im Analysegerät (200) zweite Daten generiert werden, die eine glykämische Antwort der Person darstellen, wobei eine Zeitreihe von Blutzuckermessungen, die von den ersten Daten dargestellt werden, mit einem Referenzwert für den ersten Blutzuckerspiegel der Person verglichen werden;
e) die zweiten Daten mit einem vorbestimmten individuellen Budget für die glykämische Antwort für die geeignete Person verglichen werden, wobei das individuelle Budget für die glykämische Antwort eine Gesamtmenge einer individuellen glykämischen Antwort darstellt, die für einen bestimmten Zeitraum zulässig ist, entsprechend einer empfohlenen Gesamtkohlenhydrataufnahme in diesem Zeitraum; und
f) Feedback entsprechend einem Ergebnis des Vergleichs der zweiten Daten mit dem vorbestimmten individuellen Budget für die glykämische Antwort an der Ausgabevorrichtung (270) bereitgestellt wird.

2. Verfahren nach Anspruch 1, **dadurch gekennzeichnet, dass** Schritt d) die Berechnung eines Werts für die Fläche unter der Kurve (AUC), insbesondere eines inkrementalen Werts für die Fläche unter der Kurve (iAUC) umfasst.

3. Verfahren nach Anspruch 2, **dadurch gekennzeichnet, dass** Schritt e) den Vergleich des berechneten Werts für die Fläche unter der Kurve (AUC), der einem vorbestimmten Zeitintervall und insbesondere 24 Stunde oder einer Woche entspricht, mit einem Referenz-AUC-Wert, der einer empfohlenen Gesamtkohlenhydrataufnahme innerhalb des vorbestimmten Zeitintervalls entspricht, umfasst.

4. Verfahren nach einem der Ansprüche 1 bis 3, **dadurch gekennzeichnet, dass** es ferner den Schritt der Bereitstellung von Empfehlungen zur Erreichung persönlicher Stoffwechselziele umfasst.

5. Verfahren nach einem der Ansprüche 1 bis 4, **dadurch gekennzeichnet, dass** das Messgerät (100; 150) ein kontinuierliches Blutzuckermessgerät, insbesondere ein implantierbarer Blutzuckersensor ist.

6. Verfahren nach einem der Ansprüche 1 bis 5, **dadurch gekennzeichnet, dass** eine Übertragung der ersten Daten an das Analysegerät (200) je nach Wert des gemessenen Blutzuckerspiegels aktiviert wird.

7. Verfahren nach einem der Ansprüche 1 bis 6, **dadurch gekennzeichnet, dass** der Schritt der Bereitstellung von Feedback an die Ausgabevorrichtung (270) eine Interaktion des Anwenders zur Kontrolle der Interpretation der zweiten Daten beinhaltet.

8. Verfahren nach einem der Ansprüche 1 bis 7, das einen Schritt der Selbstkalibrierung für das Messgerät (100; 150) umfasst, die den Schritt der Festlegung des Referenzwerts für den Nüchternblutzuckerspiegel der Person umfasst.

9. Verfahren nach Anspruch 8, **dadurch gekennzeichnet, dass** der Schritt der Selbstkalibrierung während Zeiträumen ohne Aufnahme von Nahrung und Getränken mit Einfluss auf den Blutzuckerspiegel durch die Person automatisch und regelmäßig, insbesondere regelmäßig über Nacht durchgeführt wird.

10. Verfahren nach Anspruch 8 oder 9, **dadurch gekennzeichnet, dass** der Schritt der Selbstkalibrierung die folgenden Unterschritte umfasst:
a) Überwachung des Blutzuckerspiegels der Person während mindestens sechs und insbesondere während mindestens acht aufeinanderfolgenden Studien ohne Aufnahme von Nahrung und Getränken;
b) Bestimmung, wann sich der Blutzuckerspiegel auf einem Nüchternwert stabilisiert hat; und
c) Mittelung eines Signals, das einer gemessenen Blutzuckerkonzentration während eines Intervalls mit größter Signalstabilität entspricht, um die Referenzdaten zu bestimmen, die dem Nüchternblutzuckerspiegel entsprechen.

11. Verfahren nach einem der Ansprüche 1 bis 10, das einen Schritt der Korrektur eines Rohsignals, das dem gemessenen Blutzuckerspiegel entspricht, gegen eine Abweichung, Signalinstabilität oder einen Systemfehler.

12. Blutzuckerüberwachungssystem zur Überwachung der glykämischen Antwort bei einer geeigneten Person und zur Erzeugung von Ernährungsfeedback; das Folgendes umfasst:
a) ein Messgerät (100; 150) mit einem Sensor zur Durchführung einer Vielzahl von aufeinanderfolgenden Messungen eines Blutzuckerspiegels bei der geeigneten Person und einem Datengenerator zur Generierung von ersten Daten, die dem gemessenen Blutzuckerwert entsprechen;
b) ein Analysegerät (200) zur Auswertung der ersten Daten; und
c) eine Ausgabevorrichtung (270) zur Bereitstellung von Feedback, **dadurch gekennzeichnet, dass**;
d) das Analysegerät (200) einen Computer umfasst, der so konfiguriert ist, dass er zweite Daten aus den ersten Daten generiert, wobei die zweiten Daten eine glykämische Antwort der Person darstellen und wobei die Generierung der zweiten Daten den Vergleich einer Zeitreihe von Blutzuckermessungen, die von den ersten Daten dargestellt werden, mit einem Referenzwert für den Nüchternblutzuckerspiegel der Person darstellen; und die zweiten Daten mit einem vorbestimmten individuellen Budget für die glykämische Antwort der geeigneten Person vergleicht, wobei das individuelle Budget für die glykämische Antwort eine Gesamtmenge der individuellen glykämischen Antwort darstellt, die für einen bestimmten Zeitraum zulässig ist, entsprechend einer empfohlenen Gesamtkohlenhydrataufnahme in diesem Zeitraum; und
e) die Ausgabevorrichtung (270) durch einen Computer gesteuert wird und Feedback entsprechend einem Ergebnis des Vergleichs der zweiten Daten mit dem vorbestimmten individuellen Budget für die glykämische Antwort bereitstellen kann.

13. System nach Anspruch 12, **dadurch gekennzeichnet, dass** das Messgerät (150) ein implantierbarer Blutzuckersensor ist.

14. System nach Anspruch 13, **dadurch gekennzeichnet, dass** das Messgerät (150) einen Speicher (180) zum vorübergehenden Speichern der ersten Daten umfasst, und dass das Messgerät und das Analysegerät (200) Übertragungskomponenten (190, 191, 240) zum Abladen der angesammelten gespeicherten ersten Daten an das Analysegerät (200) umfassen, insbesondere nachdem das Messgerät (150) vom Körper der Person entfernt wurde.

15. System nach einem der Ansprüche 12 bis 14, **dadurch gekennzeichnet, dass** das Messgerät (100) und das Analysegerät (200) Übertragungskomponente (140, 141, 240, 241) zum Übertragen der ersten Daten vom Messgerät zum Analysegerät (200) über eine drahtlose Verbindung umfassen, ferner **dadurch gekennzeichnet, dass** das Analysegerät einen Speicher zum Speichern der erhaltenen ersten Daten umfasst.

16. System nach einem der Ansprüche 12 bis 15, **gekennzeichnet durch** ein Handgerät (200), das das Analysegerät und die Ausgabevorrichtung umfasst.

17. System nach einem der Ansprüche 12 bis 16, **dadurch gekennzeichnet, dass** das Analysegerät einen Speicher zum Speichern des vorbestimmten individuellen Budgets für die glykämische Antwort für die geeignete Person umfasst.

18. System nach einem der Ansprüche 12 bis 17, **dadurch gekennzeichnet, dass** das Messgerät und/oder das Analysegerät einen Computer und/oder einen analogen elektronischen Kreislauf zum Filtern von Rauschen und/oder zum Korrigieren eines Rohsignals umfasst, das dem gemessenen Blutzuckerspiegel entspricht, gegen eine Abweichung, Signalinstabilität oder einen Systemfehler.

19. System nach einem der Ansprüche 12 bis 18, **dadurch gekennzeichnet, dass** das Analysegerät und/oder die Ausgabevorrichtung ein Anwendereingabegerät (260) umfasst und dass es so konstruiert und programmiert ist, dass ein Anwender die Interpretation der zweiten Daten mit dem Eingabegerät (260) kontrollieren kann.

20. System nach einem der Ansprüche 12 bis 19, **dadurch gekennzeichnet, dass** das Analysegerät einen Speicher zum Speichern der zweiten Daten und von vom Anwender spezifizierten Informationen umfasst.

21. Computerprogrammprodukt mit einem Programmcode, der bei Ausführung auf einem Analysegerät die folgenden Schritte durchführt:
a) Generierung von zweiten Daten, die eine glykämische Antwort einer geeigneten Person darstellen, aus den ersten Daten, wobei eine Zeitreihe von Blutzuckermessungen an einer geeigneten Person, die von den ersten Daten dargestellt werden, mit einem Referenzwert für den Nüchternblutzuckerspiegel der Person verglichen wird;
b) Vergleich der zweiten Daten mit einem vorbestimmten individuellen Budget für die glykämische Antwort für die geeignete Person, wobei das individuelle Budget für die glykämische Antwort eine Gesamtmenge einer individuellen glykämischen Antwort darstellt, die für einen bestimmten Zeitraum zulässig ist, entsprechend einer empfohlenen Gesamtkohlenhydrataufnahme in diesem Zeitraum; und
c) Generierung von zumindest einer Menge, die mit dem Ernährungsfeedback in Zusammenhang steht, auf Grundlage des Ergebnisses des Vergleichs der zweiten Daten mit dem vorbestimmten individuellen Budget für die glykämische Antwort.

## Revendications

1. Procédé de surveillance d'une réponse métabolique individuelle et de production d'une rétroaction nutritionnelle, qui comporte la surveillance d'une réponse glycémique chez un sujet qualifié, comprenant les étapes qui consistent :
a) à effectuer consécutivement une pluralité de mesures d'un taux de glucose chez le sujet qualifié à l'aide d'un dispositif de mesure (100 ; 150) ;
b) à produire, dans le dispositif de mesure (100 ; 150), des premières données qui correspondent au taux de glucose mesuré ;
c) à transmettre les premières données à un dispositif d'analyse (200) et à fournir une rétroaction sur un dispositif de sortie (270) ;
**caractérisé en ce que** :
d) dans le dispositif d'analyse (200), de secondes données sont produites, lesquelles représentent une réponse glycémique du sujet qui comporte la comparaison d'une série temporelle de mesures de glucose représentées par les premières données avec une valeur de référence pour le taux de glucose à jeun du sujet ;
e) les secondes données sont comparées avec un bilan de réponse glycémique individuelle prédéterminé pour le sujet qualifié, le bilan de réponse glycémique individuelle représentant une quantité totale de réponse glycémique individuelle permise pendant une certaine période qui correspond à un apport total recommandé d'hydrates de carbone au cours de cette période ; et
f) une rétroaction est fournie qui correspond à un résultat de la comparaison des secondes données avec le bilan de réponse glycémique individuelle prédéterminé sur le dispositif de sortie (270).

2. Procédé selon la revendication 1, **caractérisé en ce que** l'étape d) comprend le calcul d'une valeur pour l'aire sous la courbe (ASC), en particulier d'une valeur pour l'aire sous la courbe incrémentale (ASCi).

3. Procédé selon la revendication 2, **caractérisé en ce que** l'étape e) comprend la comparaison de la valeur calculée pour l'aire sous la courbe (ASC) qui correspond à un intervalle de temps prédéterminé, en particulier 24 heures ou une semaine, à une valeur ASC de référence qui correspond à un apport total recommandé d'hydrates de carbone pendant l'intervalle de temps prédéterminé.

4. Procédé selon l'une quelconque des revendications 1 à 3, **caractérisé en ce qu'**il comprend en outre l'étape consistant à fournir des suggestions relativement à la manière d'atteindre des objectifs métaboliques personnels.

5. Procédé selon l'une quelconque des revendications 1 à 4, **caractérisé en ce que** le dispositif de mesure (100 ; 150) est un dispositif de surveillance du glucose en continu, en particulier un capteur de glucose implantable.

6. Procédé selon l'une quelconque des revendications 1 à 5, **caractérisé en ce qu'**une transmission des premières données au dispositif d'analyse (200) est activée selon une valeur du taux de glucose mesuré.

7. Procédé selon l'une quelconque des revendications 1 à 6, **caractérisé en ce que** l'étape qui consiste à fournir une rétroaction sur le dispositif de sortie (270) comporte une interaction de l'utilisateur pour contrôler l'interprétation des secondes données.

8. Procédé selon l'une quelconque des revendications 1 à 7, comprenant une étape d'auto-étalonnage du dispositif de mesure (100 ; 150), comprenant l'étape d'établissement de la valeur de référence pour le taux de glucose à jeun du sujet.

9. Procédé selon la revendication 8, **caractérisé en ce que** l'étape d'auto-étalonnage est automatiquement et régulièrement effectuée pendant des périodes sans ingestion de nourriture et de boissons modifiant le glucose par le sujet, en particulier régulièrement pendant la nuit.

10. Procédé selon la revendication 8 ou 9, **caractérisé en ce que** l'étape d'auto-étalonnage comprend les sous-étapes suivantes :
a) la surveillance du taux de glucose du sujet pendant au moins six, en particulier au moins huit, heures consécutives sans ingestion de nourriture ou de boissons ;
b) la détermination du moment où le glucose s'est stabilisé au taux à jeun ; et
c) le moyennage d'un signal correspondant à une concentration de glucose mesurée pendant un intervalle de stabilité maximale de signal pour établir les données de référence qui correspondent au taux de glucose à jeun.

11. Procédé selon l'une quelconque des revendications 1 à 10, comprenant une étape de correction d'un signal brut correspondant au taux de glucose mesuré par rapport à une dérive, une instabilité du signal ou une erreur du système.

12. Système de surveillance du glucose pour la surveillance d'une réponse glycémique chez un sujet qualifié et pour la production d'une rétroaction nutritionnelle, comprenant
a) un dispositif de mesure (100 ; 150) comprenant un capteur pour effectuer consécutivement une pluralité de mesures d'un taux de glucose chez le sujet qualifié et comprenant un générateur de données pour produire des premières données qui correspondent au taux de glucose mesuré ;
b) un dispositif d'analyse (200) pour l'analyse des premières données ; et
c) un dispositif de sortie (270) pour fournir une rétroaction ;
**caractérisé en ce que** :
d) le dispositif d'analyse (200) comprend un ordinateur configuré pour générer des secondes données à partir des premières données, les secondes données représentant une réponse glycémique du sujet et la production des secondes données comparant une série temporelle de mesures de glucose représentées par les premières données avec une valeur de référence pour le taux de glucose à jeun du sujet, et pour comparer les secondes données avec un bilan de réponse glycémique individuelle prédéterminé pour le sujet qualifié, le bilan de réponse glycémique individuelle représentant une quantité totale de réponse glycémique individuelle permise pendant une certaine période qui correspond à un apport total recommandé d'hydrates de carbone au cours de cette période ; et
e) le dispositif de sortie (270) est commandé par ordinateur et arrangé pour fournir une rétroaction qui correspond à un résultat de la comparaison des secondes données avec le bilan de réponse glycémique individuelle prédéterminé.

13. Système selon la revendication 12, **caractérisé en ce que** le dispositif de mesure (150) est un capteur de glucose implantable.

14. Système selon la revendication 13, **caractérisé en ce que** le dispositif de mesure (150) comprend un dispositif de stockage (180) pour stocker temporairement les premières données et **en ce que** le dispositif de mesure et le dispositif d'analyse (200) comprennent des composants de transmission (190, 191, 240) pour le transfert des premières données stockées accumulées au dispositif d'analyse (200), en particulier après que le dispositif de mesure (150) a été retiré du corps du sujet.

15. Système selon l'une quelconque des revendications 12 à 14, **caractérisé en ce que** le dispositif de mesure (100) et le dispositif d'analyse (200) comprennent des composants de transmission (140, 141, 240, 241) pour transmettre les premières données du dispositif de mesure au dispositif d'analyse (200) par un lien sans fil, **caractérisé en outre en ce que** le dispositif d'analyse comprend un dispositif de stockage pour stocker les premières données reçues.

16. Système selon l'une quelconque des revendications 12 à 15, **caractérisé par** un dispositif portatif (200) comprenant le dispositif d'analyse et le dispositif de sortie.

17. Système selon l'une quelconque des revendications 12 à 16, **caractérisé en ce que** le dispositif d'analyse comprend un dispositif de stockage pour stocker le bilan de réponse glycémique individuelle prédéterminé pour le sujet qualifié.

18. Système selon l'une quelconque des revendications 12 à 17, **caractérisé en ce que** le dispositif de mesure et/ou le dispositif d'analyse comprennent un ordinateur et/ou un circuit électronique analogique pour filtrer le bruit et/ou pour corriger un signal brut correspondant au taux de glucose mesuré par rapport à une dérive, une instabilité du signal ou une erreur du système.

19. Système selon l'une quelconque des revendications 12 à 18, **caractérisé en ce que** le dispositif d'analyse et/ou le dispositif de sortie comprennent un périphérique d'entrée pour l'utilisateur (260) et **en ce qu'**il est conçu et programmé de telle façon qu'un utilisateur peut contrôler l'interprétation des secondes données en utilisant le périphérique d'entrée (260).

20. Système selon l'une quelconque des revendications 12 à 19, **caractérisé en ce que** le dispositif d'analyse comprend un dispositif de stockage pour stocker des secondes données ainsi que des renseignements particuliers à l'utilisateur.

21. Produit-programme informatique comprenant un code de programme qui, lorsqu'exécuté dans un dispositif d'analyse, effectue les étapes suivantes :
a) production de secondes données, lesquelles représentent une réponse glycémique d'un sujet qualifié à partir de premières données, comportant la comparaison d'une série temporelle de mesures de glucose chez un sujet qualifié, représentées par les premières données, avec une valeur de référence pour le taux de glucose à jeun du sujet ;
b) comparaison des secondes données avec un bilan de réponse glycémique individuelle prédéterminé pour le sujet qualifié, le bilan de réponse glycémique individuelle représentant une quantité totale de réponse glycémique individuelle permise pendant une certaine période qui correspond à un apport total recommandé d'hydrates de carbone au cours de cette période ; et
c) production d'au moins une quantité se rapportant à une rétroaction nutritionnelle basée sur le résultat de la comparaison des secondes données avec le bilan de réponse glycémique individuelle prédéterminé.
